Europäisches Patentamt

European Patent Office

Office européen des brevets

(11) Numéro de publication: **0 150 155**
**B1**

(12) **FASCICULE DE BREVET EUROPEEN**

(45) Date de publication du fascicule du brevet:
**07.01.88**

(21) Numéro de dépôt: **85420008.6**

(22) Date de dépôt: **15.01.85**

(51) Int. Cl.⁴: **A 61 M 1/30**

(54) Rein artificiel à aiguille unique.

(30) Priorité: **18.01.84 FR 8400932**

(43) Date de publication de la demande:
**31.07.85 Bulletin 85/31**

(45) Mention de la délivrance du brevet:
**07.01.88 Bulletin 88/1**

(84) Etats contractants désignés:
**BE CH DE GB IT LI NL SE**

(56) Documents cités:
**DE - A - 2 644 062**
**DE - A - 3 046 162**
**FR - A - 2 459 050**
**US - A - 4 153 554**
**US - A - 4 371 385**

(73) Titulaire: **HOSPAL INDUSTRIE, 7, Avenue Lionel Terray,**
**F-69330 Meyzieu (FR)**

(72) Inventeur: **Chevallet, Jacques, 8, route de Ternay,**
**F-69360 Serezin du Rhone (FR)**

(74) Mandataire: **Gauckler, Jacques et al, Service Brevets**
**HOSPAL HOSPAL C.O.T. B.P. 21, F-69881 Meyzieu**
**Cedex (FR)**

## Description

La présente invention concerne un perfectionnement apporté à un rein artificiel comportant un circuit extracorporel de sang relié au réseau artério-veineux d'un patient par un accès unique tel que aiguille ou catheter, en forme de Y ou de T. Elle concerne plus spécialement un tel rein artificiel, dont le circuit extracorporel de sang comporte un hémodialyseur encadré par deux pompes de circulation du sang.

Des reins artificiels de ce type sont déjà connus: ils présentent l'avantage par rapport aux reins traditionnels, reliés aux patients par deux aiguilles indépendantes de ne nécessiter à chaque traitement qu'un accès unique au réseau artério-veineux du patient, ce qui est considérablement moins traumatisant.

Ainsi, par exemple, la demande de brevet allemande DE-A-2644062 décrit un tel type de rein artificiel permettant en outre de prélever des quantités prédéterminées d'eau à partir du sang par ultrafiltration à travers la membrane du dialyseur.

On sait que dans les reins artificiels à aiguille unique, le sang est déplacé dans un sens, puis dans le sens opposé, soit du patient vers le circuit extracorporel, soit de ce circuit vers le patient, alternativement, à travers une aiguille ou au moins un passage unique. Il en résulte que dans l'hémodialyseur, quels que soient les systèmes amortisseurs de pulsations utilisés, le sang ne s'écoule pas selon un débit uniforme, mais selon un débit que nous qualifierons ici de pulsé. Si l'on se réfère maintenant au débit moyen de sang, par exemple au débit horaire traversant un hémodialyseur et si l'on compare l'efficacité de dialyses, c'est-à-dire les clairances obtenues, par exemple sur l'urée ou sur la créatinine, avec les mêmes débit horaires de sang, soit avec un rein artificiel à aiguille unique, soit avec un rein artificiel classique à double aiguille, on observe par des mesures précises une perte de clairance qui peut atteindre 4% environ au détriment du rein artificiel à aiguille unique, toutes autres conditions égales par ailleurs. Cette perte de clairance est d'ailleurs d'autant plus nette que l'on opère avec des débits moyens de sang plus élevés, ce qui correspond aux possibilités et aux tendances actuelles.

On peut aussi mesurer une perte de clairance semblable, si l'on soumet le liquide de dialyse seul à un régime pulsé, au lieu d'un régime à débit uniforme, toutes autres conditions égales par ailleurs, le débit sang étant alors en particulier maintenu sensiblement uniforme.

Il semble donc que le fait de remplacer dans un hémodialyseur un débit uniforme par un débit pulsé qu'il s'agisse du sang ou du liquide de dialyse, soit préjudiciable à l'efficacité de la dialyse.

Or, selon la présente invention, il a été trouvé de manière tout à fait surprenante, que l'on pouvait procéder à des traitements par reins artificiels à aiguille unique et trouver des conditions d'efficacité améliorées, par exemple en ce qui concerne la clairance en urée et en créatinine, pouvant être aussi bonnes que celles obtenues avec des reins artificiels classiques à double aiguille, si l'on mettait en œuvre des moyens permettant de pulser simultanément à la fois le sang et le liquide de dialyse dans l'hémodialyseur.

L'objet principal de la présente invention est donc de proposer un rein artificiel à aiguille unique qui ne présente pas de perte de clairance, par rapport à un rein artificiel classique à double aiguille, à débits horaires de sang et de liquide de dialyse comparables, en particulier avec des débits moyens de sang élevés.

Un autre objet de la présente invention est de proposer un rein artificiel à aiguille unique dont le fonctionnement soit simple, économique, automatique et fiable.

D'autres objets de la présente invention apparaîtront au cours de la description qui va suivre.

Il a donc maintenant été trouvé et ceci fait l'objet de la présente invention, à la réalisation de laquelle a collaboré Monsieur Jacques Chevallet un rein artificiel comprenant un hémodialyseur qui est divisé en deux compartiments par une membrane semi-perméable permettant la dialyse et l'ultrafiltration du sang dont le premier compartiment est un élément d'un circuit extracorporel de sang du type à aiguille unique comportant deux pompes de circulation qui fonctionnent de manière séquentielle et engendrent des pulsations du sang et le second compartiment est un élément d'un circuit de liquide de dialyse qui circule généralement à contre-courant du sang, ledit circuit de liquide de dialyse comportant des moyens pour moduler périodiquement le débit dudit liquide de dialyse dans ledit hémodialyseur, entre des valeurs minimum et maximum.

Ce rein artificiel est caractérisé en ce qu'il comporte une servo-commande pour asservir lesdits moyens pour moduler le débit dudit liquide de dialyse à un organe sensible aux pulsations du sang, de sorte que les débits de sang et de liquide de dialyse sont simultanément au maximum et au minimum dans l'hémodialyseur, lesdits moyens pour moduler le débit dudit liquide de dialyse agissant selon une fréquence sensiblement constante inférieure à 1 Hertz.

Selon la présente invention, on a trouvé qu'il est avantageux de substituer à un débit régulier du liquide de dialyse un débit modulé sur le rythme des séquences d'écoulement du sang dans l'hémodialyseur, imposées par l'usage de l'aiguille unique. On a donc trouvé un nouveau rein artificiel à aiguille unique, remarquable en ce qu'il comporte des moyens pour moduler périodiquement le débit du liquide de dialyse. Ces moyens peuvent être commandés par tout dispositif connu en soi de servo-commande, ce dispositif étant relié à un organe sensible aux pulsations du sang imposées par l'usage de l'aiguille unique. Cet organe est disposé dans le circuit extracorporel de sang, de préférence à proximité immédiate de l'hémodialyseur.

Par pulsation de sang, on entend ici tout pulsation de fréquence relativement basse, inférieure à un hertz et de préférence toute pulsation comprise entre 0,05 et 1 hertz. Les pulsations de sang engendrées par l'usage de l'aiguille unique sont en effet avantageusement comprises entre 0,1 et 0,4 hertz. Selon le système de régulation du mouvement des pompes de circulation du sang, la fréquence de ces pulsations est, soit rigoureusement constante, car imposée, soit le plus souvent sensiblement constante, en fonction des légères fluctuations possibles des variations de pression de sang mesurées dans le circuit extracorporel de sang à proximité immédiate de l'hémodialyseur.

On remarque que ces pulsations provoquées par l'usage de l'aiguille unique, ont des fréquences sensiblement plus basses que celles provoquées par toute autre cause, telle que battement cardiaque ou pompage par pompes à diaphragme, à piston ou péristaltique. On note en outre que l'amplitude du débit de sang plusé par exemple par une pompe péristaltique ne varie que de ± 5% environ autour du débit moyen, alors que la variation d'amplitude du débit de sang séquentiel imposé par l'usage de l'aiguille unique est généralement supérieure à ± 30% autour du débit moyen dans l'hémodialyseur, et ceci malgré l'emploi d'amortisseur de pulsations. Les pulsations du sang auxquelles se réfère la présente invention se distinguent donc fondamentalement et sans ambiguïté d'autres types de pulsations que l'on pourrait rencontrer. Et si l'on a conservé ici le terme de pulsations, c'est parce qu'il a déjà été largement utilisé dans les différents sens indiqués.

Selon l'invention, on a ainsi observé que les meilleures clairances, c'est-à-dire la meilleure efficacité des échanges de matières à travers la membrane de l'hémodialyseur, sont obtenues lorsqu'on module le débit de liquide de dialyse en phase par rapport au débit de sang dans l'hémodialyseur. On synchronise donc les débits respectifs du sang et du liquide de dialyse afin qu'ils atteignent simultanément leur maximum, puis leur minimum et ceci périodiquement, selon le rythme imposé au sang par l'usage de l'aiguille unique, dans l'hémodialyseur.

Le sang et le liquide de dialyse peuvent traverser l'hémodialyseur à co-courants ou à courants croisés, mais le plus souvent ils le traversent à contre-courant.

La pression transmembranaire dans l'hémodialyseur, c'est-à-dire la différence des pressions instantanées de part et d'autre de la membrane, dans les compartiments parcourus respectivement par le sang et par le liquide de dialyse, n'est généralement pas sensiblement affectée par la modulation du débit du liquide de dialyse selon la présente invention. On sait en effet que la pression transmembranaire est responsable du débit d'ultrafiltrat qui s'écoule du sang dans le liquide de dialyse. Par exemple la pression transmembranaire n'est notamment pas modifiée dans un rein artificiel à circuit de dialyse bouclé sur lui-même, isolé de l'atmosphère et d'où l'ultrafiltrat est imposé volumétriquement.

Il en résulte que, contrairement à ce qu'on aurait normalement pu craindre dans des hémodialyseurs du type à membrane plane où le sang et le liquide de dialyse se déplacent à contre-courant en films très minces, la membrane ne subit pratiquement pas de déformation susceptible de s'opposer à l'avancement l'un vers l'autre des flux maximum de sang et de liquide de dialyse. Seuls les débits instantanés, donc les vitesses instantanées de déplacement du liquide de dialyse à la surface de la membrane sont essentiellement modifiés.

Selon l'invention, on a observé que l'on a avantage à moduler les débits du liquide de dialyse entre deux valeurs limites prédéterminées, une valeur minimum et une valeur maximum. Ces valeurs limites sont choisies, la valeur minimum assez basse pour procurer une économie substantielle de liquide de dialyse et la valeur maximum assez élevée pour atteindre une efficacité nettement améliorée des échanges. Ainsi on peut moduler le débit du liquide de dialyse dans un rein artificiel à aiguille unique entre des valeurs limites comprises pratiquement entre 100 et 1000 ml/min et de préférence entre 200 et 800 ml/min. A titre d'exemple, on a observé que des valeurs limites de 300 et 700 ml/min conviennent bien. A ces valeurs limites correspondent des débits moyens de sang habituellement obtenus sur rein artificiel à aiguille unique, par exemple généralement compris entre 200 et 400 ml/min. Bien que la modulation du débit de liquide de dialyse puisse se faire en fonction du temps selon toute loi connue, il est avantageux d'atteindre rapidement les débits minimum et maximum et de les maintenir sensiblement constants aussi longtemps que possible.

La compréhension de rein artificiel selon l'invention sera facilitée par les figures ci-jointes qui illustrent, à titre d'exemples, schématiquement et sans échelle déterminée, divers modes de réalisation.

La fig. 1 représente, pour comparaison, un rein artificiel du type à aiguille unique, selon l'art antérieur connu;

les fig. 2 à 5 représentent divers modes de réalisation du rein artificiel selon la présente invention.

Pour plus de commodité, les éléments homologues des reins artificiels représentés dans les différentes figures sont désignés par les mêmes numéros.

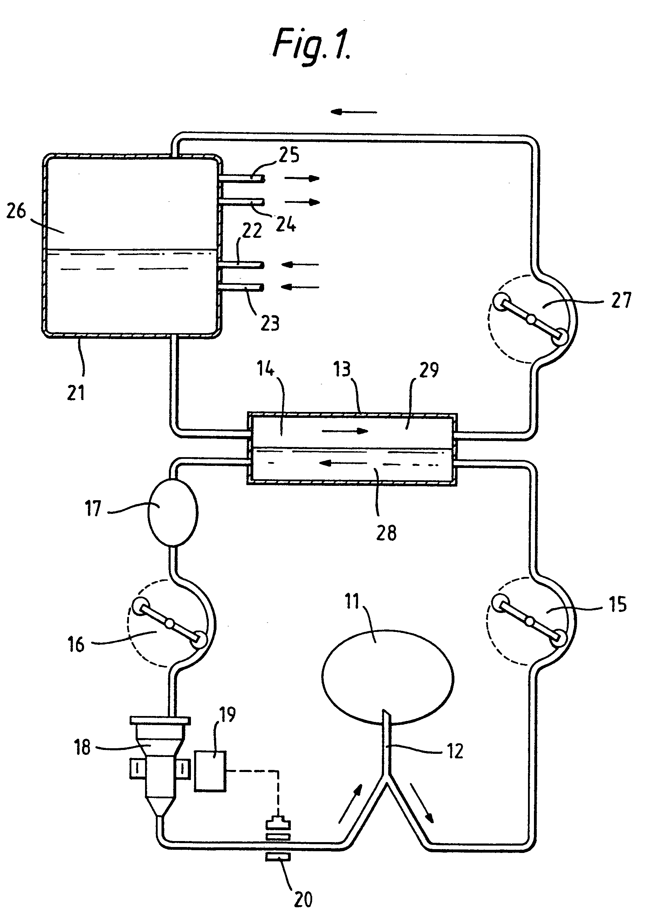

En se référant d'abord à la fig. 1, on voit que le sang est prélevé au patient (11) au moyen d'un accès unique (12) de tout type connu en soi, tel qu'une aiguille ou un cathéter, en forme de Y ou de T. Le sang parcourt un circuit extracorporel composé essentiellement d'un hémodialyseur (13) équipé d'une membrane sélectivement perméable (14) permettant la dialyse et l'ultrafiltration du sang, de préférence de deux pompes de circulation du sang (15) et (16), d'accessoires divers dont seuls les principaux ont été représentés et de lignes de liaison entre ces divers dispositifs. Comme accessoires, on ne mentionnera ici qu'un amortisseur de pulsations (17), utilisé de préférence à 1 ou 2 exemplaires, à proximité immédiate de l'hémodialyseur, notamment lorsqu'il est du type à fibres creuses; un débulleur (18) comportant un filtre (non représenté) un détecteur de bulles d'air (19) qui commande prioritairement la fermeture automatique d'une pince occlusive (20) disposée sur la ligne de retour reliant directement le débulleur à l'aiguille unique (12).

Le circuit de liquide de dialyse peut être de tout type connu, notamment ouvert sur une évacuation à l'égout, ou fermé sur lui-même, avec ou non recirculation du liquide de dialyse dans l'hémodialyseur et/ou mélange ou non du liquide usagé avec le liquide frais dans le réservoir (21). La fig. 1 représente symboliquement un tel circuit de liquide de dialyse. Le réservoir (21) comporte des moyens d'introduction dans un rapport prédéterminé d'eau (22) et de concentré de dialyse (23), ainsi que des moyens d'évacuation de l'axcès de liquide de dialyse usagé (24) et de l'ultrafiltrat (25). Un diaphragme déformable (26) peut par exemple isoler le liquide de dialyse usagé du liquide frais. Une pompe (27) disposée généralement à l'aval de l'hémodialyseur, assure une circulation régulière du liquide de dialyse, le plus souvent à contre-courant du sang. L'hémodialyseur (13) est en effet divisé par la membrane (14) en deux compartiments, le compartiment (28) est un élément du circuit extracorporel de sang et le compartiment (29) est un élément du circuit du liquide de dialyse.

Le fonctionnement de ce type de rein artificiel est séquentiel, le sang parcourant l'aiguille unique (12), alternativement du patient (11) vers l'hémodialyseur (13), puis, de celui-ci vers le patient. Les pompes à sang (15, 16) ont donc généralement un fonctionnement discontinu et décalé dans le temps. La régulation de leur mouvement s'effectue soit par programmation des durées de fonctionnement respectives, soit de préférence, en fonction de la pression du sang entre les deux pompes, pour éviter tout écart excessif de pression, par exemple à l'aide d'un capteur de pression placé à proximité de l'hémodialyseur, de préférence dans l'amortisseur de pression (17). Le fonctionnement d'une seule pompe de circulation de sang peut être commandé d'une manière analogue.

Tous les éléments du rein artificiel décrits en relation avec la fig. 1 sont bien connus et ne seront donc pas décrits ici plus en détails.

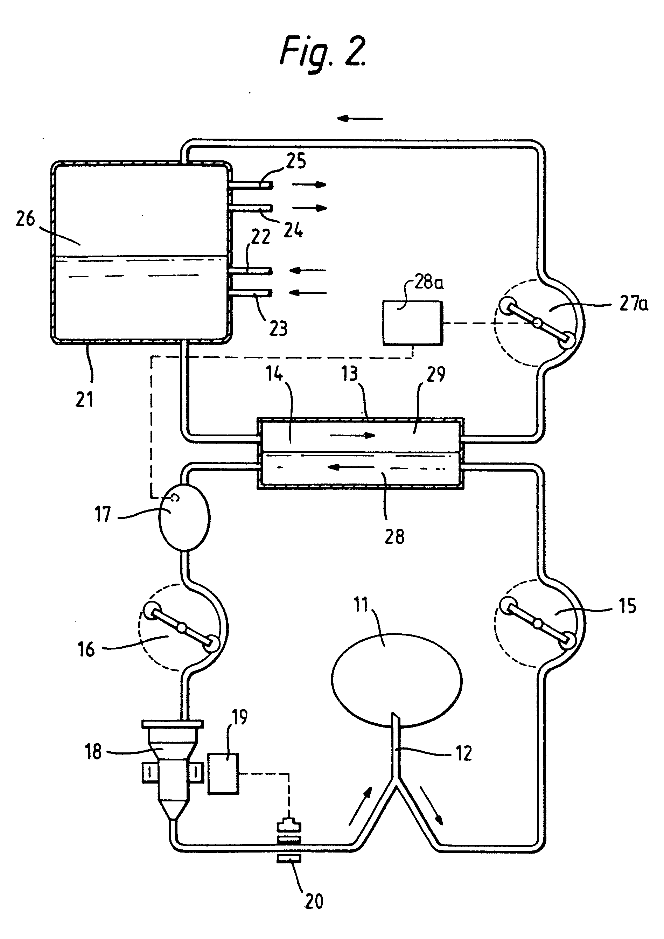

La fig. 2 représente un mode de réalisation préférentiel de la présente invention. On ne redécrit pas les éléments de ce rein artificiel à aiguille unique qui sont homologues à ceux de la fig. 1. On souligne seulement les différences essentielles entre le rein selon l'invention, représenté fig. 2 et suivantes, et le rein connu représenté fig. 1.

Selon la fig. 2, la pompe de circulation du liquide de dialyse (27a) est une pompe volumétrique à plusieurs vitesses. Elle est entraînée par un moteur qui peut commander soit une boîte de vitesses, par exemple à deux rapports différents, soit un variateur de vitesses qui peut périodiquement entraîner la pompe à des vitesses comprises entre des valeurs maximum et minimum prédéterminées. Ce groupe motopompe constitue ainsi un moyen pour moduler périodiquement le débit du liquide de dialyse dans l'hémodialyseur entre deux valeurs, minimum et maximum.

Le dispositif amortisseur de pulsations (17) disposé sur le circuit extracorporel de sang, par exemple immédiatement à l'aval de l'hémodialyseur (13), possède un capteur de la pression du sang (non repésenté) qui est un organe sensible aux pulsations du sang et qui transmet donc les valeurs instantanées de cette pression à tout moment au dispositif de servo-commande (28a). Lorsque les valeurs de la pression du sang franchissent des limites prédéterminées, la servo-commande (28a) agit automatiquement sur le groupe motopompe (27a) pour commuter sa vitesse sur l'une des vitesses minimum ou maximum prédéterminées. Compte tenu du type d'hémodialyseur, de l'amortisseur de pulsations et des caractéristiques de déformabilité sous l'effet des variations de pression du sang du circuit extracorporel de sang, le technicien étalonne expérimentalement, une fois pour toutes et par quelques essais, les valeurs de la pression du sang mesurées par le capteur de pression qui déterminent le moment optimum pour déclencher le changement de vitesse de la pompe (27a).

Le rein artificiel à aiguille unique selon la fig. 2 possède donc un hémodialyseur traversé à contre-courant par des débits pulsés en phase de sang et de liquide de dialyse. Les débits maximum et minimum traversent simultanément l'hémodialyseur. Il en résulte une efficacité des échanges sensiblement améliorée que se vérifie notamment au niveau des clairances mesurées, par exemple sur l'urée, la créatinine, l'acide urique et les phosphates.

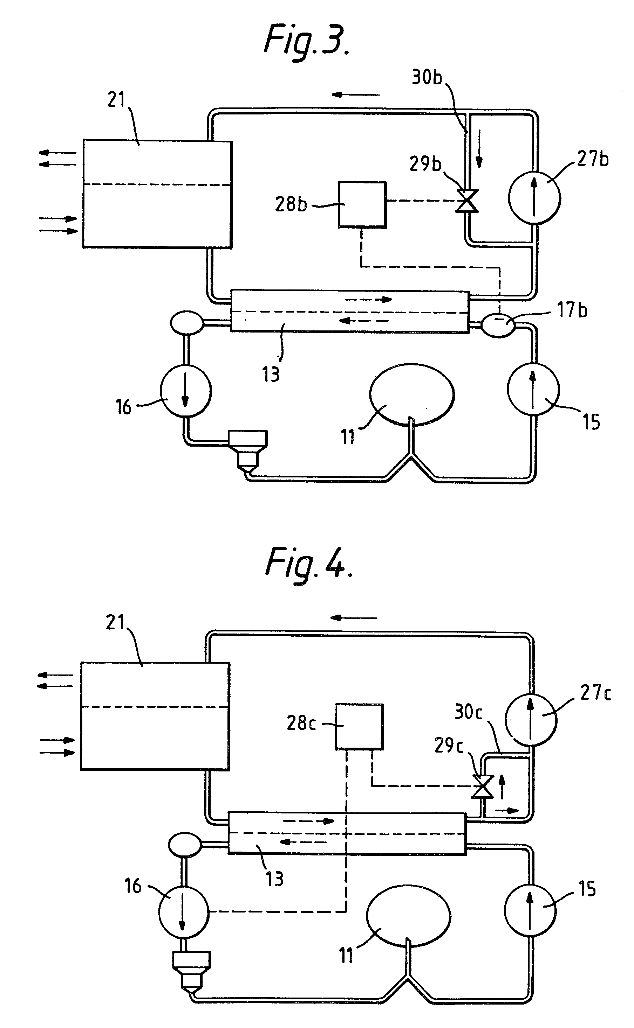

Les fig. 3 et 4 représentent deux autres modes de réalisation également avantageux de la présente invention. Les moyens pour moduler le débit de liquide de dialyse sont constitués par une canalisation de dérivation (30b, 30c) disposée en parallèle par rapport au conduit principal et par une vanne (29b, 29c) munie d'un obturateur, disposée sur cette canalisation de dérivation. La position, ouverte ou fermée, de l'obturateur de la vanne (29b, 29c) est commandée par la servo-commande (28b, 28c). Celle-ci peut être actionnée soit par un capteur de pression monté par exemple dans l'amortisseur de pulsations (17b) placé immédiatement à l'amont de l'hémodialyseur (13), soit par la mise en mouvement ou par l'arrêt du mouvement de la pompe de circulation du sang, par exemple de la pompe aval (16).

Selon la fig. 3, lé conduit de dérivation (30b) est placé en parallèle avec la pompe de circulation (27b). Celle-ci déplace le liquide de dialyse usagé en permanence de l'hémodialyseur (13) vers le réservoir (21) selon le débit maximum souhaité. Pendant la durée de l'ouverture de la vanne (29b) une partie de ce liquide est renvoyé de l'aval à l'amont de la pompe (27b) qui de ce fait n'envoie au réservoir (21) que le débit minimum prédéterminé. Les flèches indiquent le sens de circulation du liquide de dialyse. Le débit de liquide de dialyse traversant l'hémodialyseur est donc modulé périodiquement entre les valeurs minimum et maximum prédéterminées.

Selon la fig. 4, le conduit de dérivation (30c) est placé en série avec la pompe de circulation (27b) de type centrifuge, par exemple en amont. Lorsque la vanne (29c) est ouverte, le liquide de dialyse est aspiré par la pompe (27c) simultanément par le conduit principal et par le conduit de dérivation (30c) – voir flèches – et la pompe (27c) fournit alors le débit maximum prédéterminé. Lorsque la vanne (29c) est fermée, le liquide de dialyse n'accède plus à la pompe (27c) que par le conduit principal et la pompe fournit alors le débit minimum souhaité.

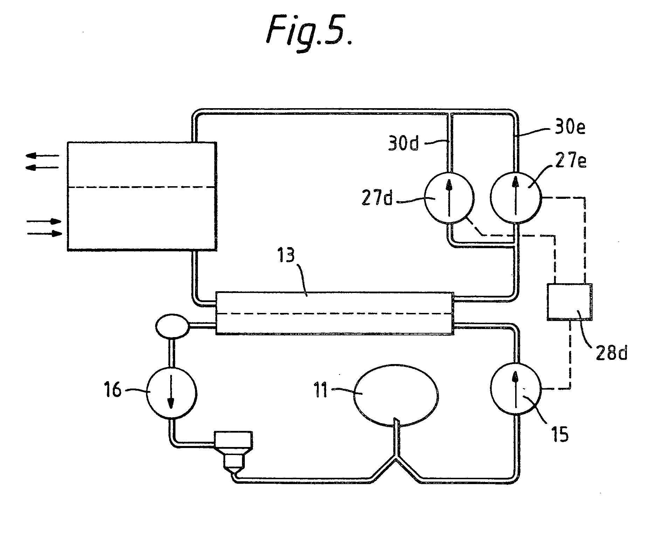

La fig. 5 représente une variante de réalisation de la présente invention. Deux pompes de circulation (27d, 27e) de liquide de dialyse sont disposées en parallèle respectivement sur des conduits (30d, 30e). La pompe (27d) seule a une capacité de débit égale au débit minimum prédéterminé et la pompe (27e) seule a une capacité de débit égale au débit maximum prédéterminé. Chacune de ces pompes est entraînée alternativement en mouvement par la servo-commande (28d), reliée par exemple à la pompe de circulation de sang amont (15).

En variante, la pompe (27d) a une capacité de débit égale au débit minimum prédéterminé et la pompe (27e) a une capacité de débit égale à la différence entre les débits maximum et minimum prédéterminés. Le fonctionnement de la pompe (27d) est alors continu, tandis que la pompe (27e), seule commandée par la servo-commande (28d) a un fonctionnement intermittent.

La présente invention peut être mise en œuvre avec tout type connu d'hémodialyseur, par exemple à plaques, à fibres creuses, ou à bobine. Le circuit extracorporel de sang peut, en outre, comporteur ou non des dispositifs amortisseurs de pulsations de tous types connus, disposés en un ou plusieurs points du circuit.

Il est à la portée du technicien de combiner entre elles ou d'aménager les différentes possibilités de moduler le débit du liquide de dialyse mentionnées ici ou d'en mettre au point d'équivalentes. Par exemple on peut actionner périodiquement une vanne placée sur le circuit du liquide de dialyse entre une position pleine ouverture et une position ouverture partielle de l'obturateur. La commande séquentielle de débit de liquide de dialyse en phase avec les pulsations du sang dans un rein artificiel à aiguille unique peut évidemment faire l'objet de nombreuses autres variantes de réalisation à la portée du technicien, sans sortir du cadre de la présente invention.

**Revendications**

1. Rein artificiel comprenant un hémodialyseur (13) qui est divisé en deux compartiments par une membrane semi-perméable (14) permettant la dialyse et l'ultrafiltration du sang dont le premier compartiment (28) est un élément d'un circuit extracorporel de sang du type à aiguille unique comportant deux pompes de circulation (15, 16) qui fonctionnent de manière séquentielle et engendrent des pulsations du sang et le second compartiment (29) est un élément d'un circuit de liquide de dialyse qui circule généralement à contre-courant du sang, ledit circuit de liquide de dialyse comportant des moyens (27a, b, c, d, e) pour moduler périodiquement le débit dudit liquide de dialyse dans ledit hémodialyseur (13) entre des valeurs minimum et maximum, caractérisé en ce qu'il comporte un servo-commande (28a, b, c, d) pour asservir lesdits moyens (27a, b, c, d, e) à un organe (15, 16, 17) sensible aux pulsations du sang, de sorte que les débits de sang et de liquide de dialyse sont simultanément au maximum et au minimum dans l'hémodialyseur, lesdits moyens (27a, b, c, d, e) agissant selon une fréquence sensiblement constante inférieure à 1 hertz.

2. Rein artificiel selon la revendication 1, caractérisé en ce que les moyens (27a, b, c, d, e) pour moduler le débit du liquide de dialyse agissent selon une fréquence sensiblement constante comprise entre 0,1 et 1 hertz.

3. Rein artificiel selon l'une quelconque des revendications précédentes, caractérisé en ce que le débit du liquide de dialyse est modulé entre 100 et 1000 ml/min.

4. Rein artificiel selon l'une quelconque des revendications précédentes, caractérisé en ce que ledit organe sensible aux pulsations du sang est une prise de pression instantanée, située sur ledit circuit extracorporel de sang, en un point (17) proche dudit hémodialyseur (13).

5. Rein artificiel selon l'une quelconque des revendications 1 à 3, caractérisé en ce que ledit organe sensible aux pulsations du sang est une pompe de circulation du sang (15, 16) sur ledit circuit extracorporel de sang.

6. Rein artificiel selon l'une quelconque des revendications précédentes, caractérisé en ce que lesdits moyens pour moduler le débit du liquide de dialyse sont constituées par une pompe de circulation de type volumétrique (27a), à plusieurs vitesses, commandée périodiquement par ladite servo-commande (28a).

7. Rein artificiel selon l'une quelconque des revendications 1 à 5, caractérisé en ce que lesdits moyens pour moduler le débit du liquide de dialyse sont constitués par une canalisation de dérivation (30b, c) disposée en parallèle par rapport au conduit principal, une vanne (29b, c) munie d'un obturateur étant disposée sur ladite canalisation de dérivation, la position dudit obturateur étant commandée périodiquement par ladite servo-commande (28b, c).

8. Rein artificiel selon l'une quelconque des revendications 1 à 5, caractérisé en ce que lesdits moyens pour moduler le débit du liquide de dialyse sont constitués par deux pompes de circulation (27d, e) disposées en parallèle dont l'une au moins est commandée périodiquement par ladite servo-commande (28d).

## Patentansprüche

1. Künstliche Niere mit einem durch eine semipermeable Membran (14) in zwei Abteile unterteilten Hämodialysator für die Dialyse und Ultrafiltration von Blut, dessen erstes Abteil (28) ein Element eines ausserhalb des Körpers gelegenen Blutkreislaufs von der Bauart mit einziger Nadel ist, der zwei Zirkulationspumpen (15, 16) aufweist, die aufeinanderfolgend arbeiten und ein Pulsieren des Bluts erzeugen, und dessen zweites Abteil (29) ein Element eines Kreislaufs für Dialyseflüssigkeit ist, die im allgemeinen im Gegenstrom zum Blut zirkuliert, wobei der Dialyseflüssigkeitskreislauf Mittel (27a, b, c, d, e) aufweist zum periodischen Modulieren der Durchsatzmenge der Dialyseflüssigkeit im Hämodialysator (13) zwischen den Minimal- und Maximalwerten, dadurch gekennzeichnet, dass sie eine Servosteuerung (28a, b, c, d) aufweist zum Regeln der Mittel (27a, b, c, d, e) nach einem auf das Pulsieren des Bluts empfindlichen Organ (15, 16, 17), so dass die Durchsatzmengen an Blut und Dialyseflüssigkeit im Hämodialysator sich gleichzeitig auf dem Maximum und Minimum befinden, wobei die Mittel (27a, b, c, d, e) gemäss einer Frequenz arbeiten, die im wesentlichen konstant und kleiner als 1 Hz ist.

2. Künstliche Niere nach Anspruch 1, dadurch gekennzeichnet, dass die Mittel (27a, b, c, d, e) zum Modulieren der Durchsatzmenge an Dialyseflüssigkeit gemäss einer im wesentlichen konstanten Frequenz zwischen 0,1 und 1 Hz wirken.

3. Künstliche Niere nach einem der vorhergehenden Ansprüche, dadurch gekennzeichnet, dass die Durchsatzmenge an Dialyseflüssigkeit zwischen 100 und 1000 ml/min moduliert wird.

4. Künstliche Niere nach einem der vorhergehenden Ansprüche, dadurch gekennzeichnet, dass das auf das Pulsieren des Bluts empfindliche Organ eine Entnahmestelle für augenblicklichen Druck ist, die sich an einem dem Hämodialysator (13) nahegelegenen Punkt am ausserhalb des Körpers gelegenen Kreislauf befindet.

5. Künstliche Niere nach einem der Ansprüche 1 bis 3, dadurch gekennzeichnet, dass das auf das Pulsieren des Bluts empfindliche Organ eine am ausserhalb des Körpers gelegenen Blutkreislauf gelegene Umwälzpumpe (15, 16) für Blut ist.

6. Künstliche Niere nach einem der vorhergehenden Ansprüche, dadurch gekennzeichnet, dass die Mittel zum Modulieren der Durchsatzmenge an Dialyseflüssigkeit durch eine Zirkulationspumpe (27a) von der volumetrischen Bauart und mit mehreren Drehzahlen gebildet ist, die durch die Servosteuerung (28a) periodisch gesteuert wird.

7. Künstliche Niere nach einem der Ansprüche 1 bis 5, dadurch gekennzeichnet, dass die Mittel zum Modulieren der Durchsatzmenge an Dialyseflüssigkeit durch eine Abzweigleitung (30b, c) gebildet sind, die sich parallel zur Hauptleitung erstreckt, wobei ein mit einem Verschlussglied versehenes Ventil (29b, c) in der Abzweigleitung angeordnet ist, wobei die Stellung des Verschlussglieds durch die Servosteuerung (28b, c) periodisch gesteuert wird.

8. Künstliche Niere nach einem der Ansprüche 1 bis 5, dadurch gekennzeichnet, dass die Mittel zum Modulieren der Durchsatzmenge an Dialyseflüssigkeit durch zwei parallel geschaltete Zirkulationspumpen (27d, e) gebildet sind, von denen wenigstens eine durch die Servosteuerung (28d) periodisch gesteuert wird.

## Claims

1. Artificial kidney comprising a blood dialyser (13) which is divided into two compartments by a semipermeable membrane (14) permitting the dialysis and the ultrafiltration of the blood, in which the first compartment (28) is a member of a blood circuit of the single-needle type, external to the body, comprising two circulation pumps (15, 16) which operate sequentially and produce blood pulsations, and the second compartment (29) is a member of a circuit for dialysis liquid which circulates generally countercurrentwise to the blood, the said circuit for dialysis liquid comprising means (27a, b, c, d, e) for modulating at regular intervals the flow rate of the said dialysis liquid in the said blood dialyser (13) between minimum and maximum values, characterized in that it comprises a servo controller (28a, b, c, d) for subjecting the said means (27a, b, c, d, e) to control by a device (15, 16, 17) which is sensitive to blood pulsations, so that the flow rates of the blood and of the dialysis liquid are simultaneously at the maximum and at the minimum in the blood dialyser, the said means (27a, b, c, d, e) operating at a substantially constant frequency of less than 1 hertz.

2. Artificial kidney according to claim 1, characterized in that the means (27a, b, c, d, e) for modulating the flow rate of the dialysis liquid operate at a substantially constant frequency of between 0.1 and 1 hertz.

3. Artificial kidney according to either of the preceding claims, characterized in that the flow rate of the dialysis liquid is modulated between 100 and 1000 ml/min.

4. Artificial kidney according to any one of the preceding claims, characterized in that the said device which is sensitive to blood pulsations is an instantaneous pressure sensor situated in the said blood circuit external to the body, at a point (17) close to the said blood dialyser (13).

5. Artificial kidney according to any one of claims 1 to 3, characterized in that the said device which is sensitive to blood pulsations is a blood circulation pump (15, 16) in the said blood circuit external to the body.

6. Artificial kidney according to any one of the preceding claims, characterized in that the said means for modulating the flow rate of the dialysis liquid consists of a multispeed circulation pump of a volumetric type (27a), controlled at regular intervals by the said servo controller (28a).

7. Artificial kidney according to any one of claims 1 to 5, characterized in that the said means for modulating the flow rate of the dialysis liquid consists of a branch line (30b, c) arranged in parallel in relation to the main conduit, a valve (29b, c) fitted with a shutter being arranged in the said branch line, the position of the said shutter being controlled at regular intervals by the said servo controller (28b, c).

8. Artificial kidney according to any one of claims 1 to 5, characterized in that the said means for modulating the flow rate of the dialysis liquid consist of two circulation pumps (27d, e) arranged in parallel, at least one of which is controlled at regular intervals by the said servo controller (28d).

# Fig.1.

# Fig. 2.

0150155

*Fig.3.*

*Fig.4.*

# Fig.5.